# EUROPÄISCHE PATENTANMELDUNG

(11) **EP 2 664 313 A1**
(43) Veröffentlichungstag der Anmeldung: **20.11.2013**
(21) Anmeldenummer: 13160394.6
(22) Anmeldetag: 21.03.2013
(51) Int. Cl.: A61F 15/00

(54) **Hygieneartikel- und/oder Pflegeartikel-Spender für im Wesentlichen flache Elemente**

(30) Priorität: 23.03.2012 DE 102012102512
(71) Anmelder: Möhn-Bock, Sabine, 72581 Dettingen/Erms (DE)
(72) Erfinder: Möhn-Bock, Sabine, 72581 Dettingen/Erms (DE)
(74) Vertreter: Kaufmann, Ursula Josefine

(57) **Zusammenfassung**

Die Erfindung betrifft einen Hygieneartikel- und/oder Pflegeartikel-Spender (10) für im Wesentlichen flache Elemente (100), umfassend ein Gehäuse (12), welches wenigstens einen Vorratsraum (14) für die Elemente (100) umschließt, sowie eine Ausgabeöffnung (16) im Gehäuse (12), durch welche die Elemente (100) einzeln entnehmbar sind. Eine Transporteinheit (40) ist vorgesehen und dazu ausgebildet, ein zu entnehmendes Element (100) zur Ausgabeöffnung (16) so zu transportieren, dass das zu entnehmende Element (100) im Benutzungsfall zumindest bereichsweise aus dem Gehäuse (12) ragt.

## Beschreibung

### Stand der Technik

Die Erfindung betrifft einen Hygieneartikel- und/oder Pflegeartikel-Spender für im Wesentlichen flache Elemente nach dem Oberbegriff des Anspruchs 1.

Aus der DE 296 12 265 U1 ist ein Spender für Slipeinlagen oder Binden bekannt. Ein Stapel derartiger Hygieneartikel ist in einem Vorratsraum eines Gehäuses bevorratet und durch eine oben auf dem Stapel aufliegende Gewichtsplatte beschwert, welche den Stapel nach unten drückt, so dass bei Entnahme eines Elements im unteren Bereich des Gehäuses der Stapel nach unten rutscht.

Die Aufgabe der Erfindung ist es, einen derartigen Spender für im Wesentlichen flache Elemente zu verbessern.

### Offenbarung der Erfindung

Die Aufgabe wird durch die Merkmale des Anspruchs 1 gelöst. Günstige Ausgestaltungen und Vorteile der Erfindung ergeben sich aus den weiteren Ansprüchen, der Beschreibung und der Zeichnung.

Die Erfindung geht aus von einem Hygieneartikel- und/oder Pflegeartikel-Spender für im Wesentlichen flache Elemente, umfassend ein Gehäuse, welches wenigstens einen Vorratsraum für die Elemente umschließt, sowie eine Ausgabeöffnung im Gehäuse, durch welche die Elemente einzeln entnehmbar sind.

Es wird vorgeschlagen, dass eine Transporteinheit vorgesehen ist, die dazu ausgebildet ist, ein zu entnehmendes Element zur Ausgabeöffnung so zu transportieren, dass das zu entnehmende Element im Benutzungsfall zumindest bereichsweise aus dem Gehäuse ragt.

Das zumindest bereichsweise aus dem Gehäuse herausragende Element kann auf einfache und hygienische Weise entnommen werden, ohne dass die Hand des Benutzers mehr als notwendig mit dem Gehäuse in Berührung kommen muss.

Die Elemente könne mit oder ohne Umverpackung vorgesehen sein.

Die Elemente können insbesondere flache Hygieneartikel sein, wie etwa Slipeinlagen oder Binden. Die Hygieneartikel können beispielsweise ohne Umverpackung einzeln zur Ausgabeöffnung transportiert werden und dort praktisch gebrauchsfertig entnommen werden.

Möglich ist auch eine Ausgabe von Elementen mit Umverpackung, insbesondere von wenigen Stücken derartiger Hygieneartikel als Vorratsset mit Umverpackung. Ragt das zu entnehmende Element bereichsweise aus dem Gehäuse, kann es bequem manuell entnommen werden.

Denkbar sind auch Pflegeartikel wie z.B. Reinigungs- und/oder Erfrischungstücher; Hygienesets mit Umverpackung, welche auch eine Mehrzahl von Komponenten enthalten können, wie zum Beispiel Einmalzahnbürsten mit oder ohne Zahncreme, Einmalrasierer mit oder ohne Rasiercreme/Rasierschaum oder dergleichen; Creme- und/oder Schaumtuben und dergleichen. Die gegebenenfalls in der Umverpackung verpackten Pflegeartikel können beispielsweise einzeln zur Ausgabeöffnung transportiert werden und dort entnommen werden. Die Umverpackung kann biegeschlaff oder biegesteif und kann aus Papier, Karton, Vlies, Kunststoff oder dergleichen sein.

Das Gehäuse kann zwei oder mehr Vorratsräume aufweisen, um in jedem Vorratsraum eine andere Art von Elementen bevorraten zu können. Es kann in jedem Vorratsraum eine Transporteinheit mit einer der Transporteinheit zugeordneten Ausgabeöffnung angeordnet sein, oder es kann eine einzige Transporteinheit mehreren Vorratsräumen zugeordnet sein.

Ferner können im Spender zusätzlich ein oder mehrere Vorratsräume für stückige Elemente vorgesehen sein, wie etwa Tampons, die durch einen separaten Transportmechanismus zu einer separaten Ausgabeöffnung transportiert und entnommen werden können. Hierdurch können eine Mehrzahl ganz verschiedenartiger flacher oder stückiger Hygieneartikel und/oder Pflegeartikel kompakt und auf hygienische Weise bereitgestellt werden. Dies ist besonders im öffentlichen Bereich vorteilhaft.

Gemäß einer günstigen Ausgestaltung kann die Transporteinheit ausgebildet sein, ein zu entnehmendes Element an wenigstens einer Seite des Elements durch Reibschluss in Richtung der Ausgabeöffnung anzutreiben. Insbesondere kann der Reibschluss durch eine Oberfläche hergestellt werden, welche einen hohen Reibwiderstand aufweist, der deutlich höher ist als der Reibwiderstand des Elements an dessen Oberfläche, welche mit der Transporteinheit antriebsmäßig in Kontakt kommt. Insbesondere kann die Oberfläche eine hohe Adhäsivität aufweisen, vorzugsweise klebrig sein und/oder aufgeraut. Zweckmäßigerweise ist die Oberfläche des darauf liegenden Elements glatter und/oder weniger adhäsiv als die Oberfläche der Transporteinheit. Das Element liegt auf der Oberfläche der Transporteinheit und wird durch die Adhäsivität der Oberfläche bei einer Bewegung der Oberfläche mitgenommen und so zur Ausgabeöffnung bewegt.

Vorteilhaft kann das zu entnehmende Element auf einer Oberfläche der Transporteinheit aufliegen und unter dem darüberliegenden Element hindurch gleiten.

Günstigerweise kann die Transporteinheit wenigstens bereichsweise eine adhäsive Oberfläche aufweisen. Hierdurch kann auf einfache Weise ein auf diesem Bereich der Oberfläche aufliegendes Element mitgenommen und zur Ausgabeöffnung bewegt werden. Insbesondere kann die Transporteinheit eine Transportrolle mit einer adhäsiven Oberfläche sein.

Gemäß einer günstigen Ausgestaltung kann die Transporteinheit zum Antreiben eines zu entnehmenden Elements eine oder mehrere Transportrollen aufweisen. Die eine oder mehrere Transportrollen können beispielsweise elektromotorisch angetrieben sein, oder auch durch einen manuell betätigbaren Mechanismus, etwa einen Kipphebel, in Bewegung versetzbar sein, wobei durch die Bewegung ein zu entnehmendes Element zur Ausgabeöffnung bewegbar ist.

Gemäß einer günstigen Ausgestaltung kann die Transporteinheit zum Antreiben eines zu entnehmenden Elements ein Transportband aufweisen. Das Transportband kann beispielsweise elektromotorisch angetrieben sein, oder auch durch einen manuell betätigbaren Mechanismus, etwa einen Kipphebel, in Bewegung versetzbar sein, wobei ein zu entnehmendes Element zur Ausgabeöffnung bewegbar ist.

Gemäß einer günstigen Ausgestaltung kann die Transporteinheit Formschlussmittel aufweisen, um ein zu entnehmendes Element durch Formschluss anzutreiben. Insbesondere kann die Transporteinheit zum Antreiben eines zu entnehmenden Elements eine oder mehrere Nadeln aufweisen, die vorteilhaft in das zu entnehmende Element einstechen und dieses dadurch selektiv bewegen können. Dies eignet sich vorteilhaft für Elemente mit wenigstens Bereichen und/oder Umverpackungen aus Textil, Vlies oder dergleichen.

Gemäß einer günstigen Ausgestaltung kann die Transporteinheit zum Antreiben eines zu entnehmenden Elements einen Drehteller aufweisen. Dies erlaubt eine Entnahme des zu entnehmenden Elements durch eine Drehbewegung des Drehtellers, auf dem das zu entnehmende Element aufliegt.

Gemäß einer günstigen Ausgestaltung kann die Transporteinheit elektromotorisch antreibbar sein. Der elektromotorische Antrieb kann durch einen Schalter in Gang gesetzt werden, oder auch berührungslos durch einen Sensor. Die elektrische Versorgung des Antriebs kann durch einen Anschluss an das Stromnetz erfolgen, oder durch Batterien oder Akkus, da der Antrieb nur kurzzeitig in Betrieb ist und das zu entnehmende Element nur über eine kurze Strecke zu transportieren ist.

Gemäß einer günstigen Ausgestaltung kann die Transporteinheit durch eine mechanisch betätigbare Hebelvorrichtung antreibbar sein. Dies ist eine besonders einfache Betätigungsart und eignet sich besonders für Einsatzbereiche, bei denen die Zahl der Nutzer klein ist, etwa in Privathaushalten.

Gemäß einer günstigen Ausgestaltung kann die Transporteinheit eine Münzaufnahme aufweisen, welche die Transporteinheit direkt oder indirekt in Gang setzt oder sperrt. Hiermit kann in öffentlich zugänglichen Bereichen ein Service z.B. für Hygieneartikel angeboten werden.

Gemäß einer günstigen Ausgestaltung kann der Vorratsraum zur Aufnahme einer Stapelanordnung der Elemente ausgebildet sein. Dies erlaubt ein einfaches Befüllen des Gehäuses mit Standard-Packungen z.B. von Slipeinlagen oder Binden.

Gemäß einer günstigen Ausgestaltung kann der Vorratsraum zur Aufnahme von Elementen als Wicklung einer Mehrzahl von Elementen ausgebildet sein. Dies erlaubt eine Bevorratung größerer Mengen von Elementen, die entsprechend für Großverbraucher konfektioniert sind.

Denkbar ist, insbesondere bei Hygieneartikeln wie Slipeinlagen oder Binden, deren Klebeseite vor deren bestimmungsgemäßen Gebrauch mit einer Abdeckfolie abgeklebt ist, an der Ausgabeöffnung einen Festhalteeinheit vorzusehen, welche die Abdeckfolie am an der Ausgabeöffnung ankommenden Ende des Elements festhält bzw. blockiert und automatisch von der Klebefläche abzieht, während das Element in die Position weiterbewegt wird, in der es aus der Ausgabeöffnung entnehmbar ist. Die Abdeckfolie bleibt zurück und fällt z.B. in einen dafür vorgesehenen Abfallbehälter im Gehäuse.

### Zeichnung

Weitere Vorteile ergeben sich aus der folgenden Zeichnungsbeschreibung. In den Zeichnungen sind Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

**Es zeigen beispielhaft:**
- Fig. 1a: ein Ausführungsbeispiel der Erfindung als Schnitt durch einen Spender mit einem Stapel von zu entnehmenden Elementen, wobei ein manuell betätigbarer Hebel Bestandteil einer Transporteinheit ist;
- Fig. 1b: in Draufsicht auf Ebene 1 b-1 b das Ausführungsbeispiel aus Figur 1a;
- Fig. 1c: ein Detail einer Transporteinheit mit Formschlussmitteln;
- Fig. 2: ein Ausführungsbeispiel mit einem Transportband;
- Fig. 3: eine schematische Darstellung einer Mehrzahl von Elementen in Stapelanordnung mit einer Niederhalte-Feder;
- Fig. 4: ein Ausführungsbeispiel der Erfindung in Draufsicht mit einem Drehteller, der Bestandteil der Transporteinheit ist.

### Ausführungsformen der Erfindung

In den Figuren sind gleiche oder gleichartige Komponenten mit gleichen Bezugszeichen beziffert. Die Figuren zeigen lediglich Beispiele und sind nicht beschränkend zu verstehen.

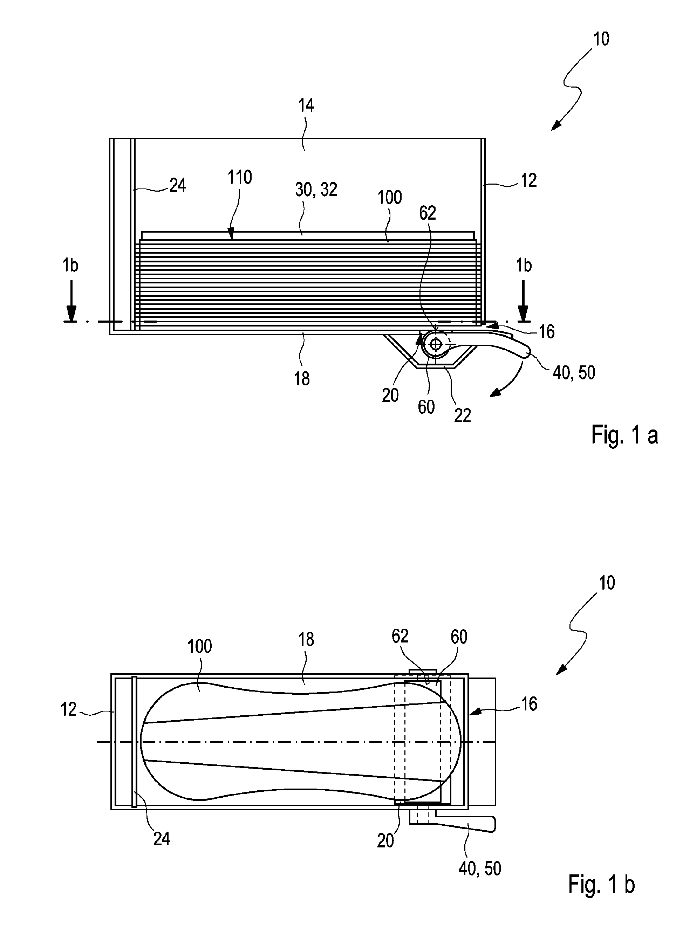

In den Figuren 1a bis 1c ist ein Ausführungsbeispiel der Erfindung als Schnitt (Figur 1a) und als Draufsicht von oben (Figur 1b) eines Hygieneartikel- und/oder Pflegeartikel-Spenders 10 mit einem Stapel 110 von zu entnehmenden Elementen 100 dargestellt, wobei ein manuell betätigbarer Hebel 50 Bestandteil einer Transporteinheit 40 ist.

Der Hygieneartikel- und/oder Pflegeartikel-Spender 10 ist für im Wesentlichen flache Elemente 100 ausgebildet und umfasst ein Gehäuse 12, welches wenigstens einen Vorratsraum 14 für die Elemente 100 umschließt. Die Elemente 100 können beispielsweise Slipeinlagen, Binden oder dergleichen sein. Der Stapel 110 kann durch eine nicht dargestellte Einfüllöffnung z.B. im oberen Bereich des Gehäuses 12 eingefüllt werden.

Das Gehäuse 12 weist in einer Seitenwand im unteren Gehäusebereich eine Ausgabeöffnung 16 auf, durch welche die Elemente 100 einzeln entnehmbar sind, wenn ein zu entnehmendes Element 100 mittels der Transporteinheit 40 zur Ausgabeöffnung 16 bewegt wird. Die Ausgabeöffnung 16 kann optional mit einer Abdeckung, beispielsweise einer Abdeckklappe, abgedeckt sein (nicht dargestellt). Die Transporteinheit 40 kann in einem am Boden 18 des Gehäuses 12 angeordneten Gehäuseteil 22 geschützt angeordnet sein.

Die Transporteinheit 40 weist in diesem Ausführungsbeispiel eine Transportrolle 60 auf, die mit dem Hebel 50 auf einer Welle gelagert ist. Wird der Hebel 50 betätigt (gekrümmter Pfeil), dreht sich die Transportrolle 60. Die Transportrolle 60 ragt teilweise durch eine Öffnung 20 an der Unterseite in das Gehäuse 12 hinein. Die Oberfläche 62 der Transportrolle 60 ist vorteilhafterweise adhäsiv ausgebildet, beispielsweise gummiert oder leicht klebrig, so dass eine Bewegung der Transportrolle 60 ein darauf aufliegendes Element 100 durch die daraus resultierende hohe Haftreibung mitnimmt. Dabei kann ausgenutzt werden, dass Elemente 100 wie Slipeinlagen oder Binden eine Rückseite mit einem Klebestreifen aufweisen, der durch eine glatten Abdeckfolie abgeklebt ist.

Das im Stapel 110 der Elemente 100 zuunterst liegende Element 100 wird dadurch zur Ausgabeöffnung 16 geschoben, während die darüberliegenden Elemente 100 nicht mit bewegt werden. Das zu entnehmende Element 100 liegt auf der Oberfläche 62 auf und gleitet unter dem darüberliegenden Element 100 hindurch. Je nachdem, wie viele Umdrehungen die Transportrolle 60 mittels Betätigung des Hebels 50 ausführt, reicht das zu entnehmende Element 100 mehr oder weniger weit aus der Ausgabeöffnung 16 und kann hygienisch entnommen werden.

Der Stapel 110 kann von oben mit einem Niederhalter 30, beispielsweise in Form einer Platte 32, nach unten gedrückt werden, um eine möglichst gleichmäßige Belastung des Stapels 110 bzw. eine Mindestlast auf die Transportrolle 60 auszuüben.

Um eine Drehung der Transportrolle 60 in Gegenrichtung (in der Zeichnung im Gegenuhrzeigersinn) zu vermeiden, kann eine Drehung in diese Richtung verhindert werden, indem die Drehrichtung mechanisch gesperrt oder die Rolle einen Freilauf für diese Richtung aufweist. Statt dem Hebel 50 kann auch eine Kurbel eingesetzt sein. Ebenso kann die Transportrolle 60 mit einem Elektromotor angetrieben sein.

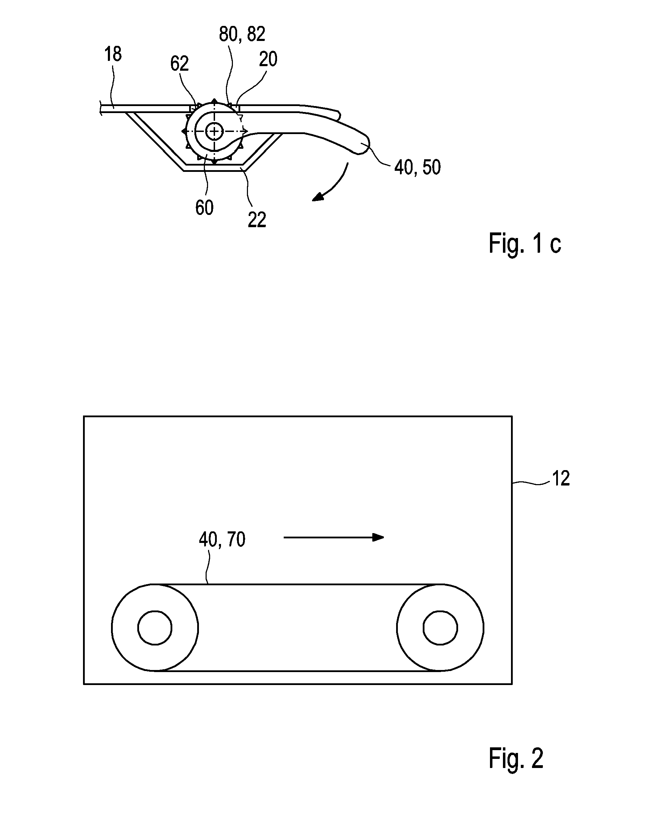

Figur 1c zeigt ein Beispiel einer Transporteinheit 40 mit einer Transportrolle 60, welche an ihrer Oberfläche Formschlussmittel 80 in Form von Nadelspitzen 82 aufweist. Wird die Transportrolle 60 gedreht, nehmen die Formschlussmittel 80 ein darauf liegendes Element 100 in Richtung zur Ausgabeöffnung 16 mit.

Sind die Elemente 100 beispielsweise handelsübliche Slipeinlagen, werden diese in das Gehäuse 12 gelegt, beispielsweise in ein separates Magazin 24. Durch Betätigen des Hebels 50 oder einen Schalter bei der elektrischen Ausführung wird die einzelne Slipeinlage über die Transportrolle 60 oder einen anderen Mechanismus durch die Ausgabeöffnung 16 bereitgestellt.

Diese wird dann vom Bediener händisch vollständig entnommen und die Schutzfolie von der Klebeschicht wie üblich entfernt. Beim Entnehmen kann der Antrieb einen Freilauf entkoppelt werden, so dass das Herausziehen kraftlos bzw. widerstandsarm erfolgen kann. Die restlichen Slipeinlagen sind geschützt und auch nicht direkt als solche erkennbar.

Mit der Erfindung wird vorteilhaft erreicht, dass handelübliche Slipeinlagen über der Hygieneartikel- und/oder Pflegeartikel-Spender 10 zum Gebrauch vereinzelt bereitgestellt werden. Dies kann durch das Einlegen dieser oder Einlegen einer Kassette oder Einstellen der entsprechenden Pappschachtel erreicht werden.

Die Erfindung eröffnet die Möglichkeit, den Hygieneartikel- und/oder Pflegeartikel-Spender 10 in einer entsprechenden Ausbaustufe in der Gastronomie einzusetzen und beispielsweise über den Einwurf von Münzen oder Wertmarken in eine entsprechende Aufnahme die Elemente 100 freizugeben.

Der Spender kann als Tisch- oder auch Wandspender in dekorativem Design ausgeführt sein und kann die üblichen Pappschachteln mit fertig konfektionierten Einlagen ablösen.
Figur 2 zeigt ein weiteres Ausführungsbeispiel einer Transporteinheit 40 mit einem Transportband 70. Dieses kann ebenso wie die Transportrolle 60 aus
Figur 1a - 1c angetrieben sein oder auch eine adhäsive oder mit Formschlussmitteln versehene Oberfläche aufweisen.

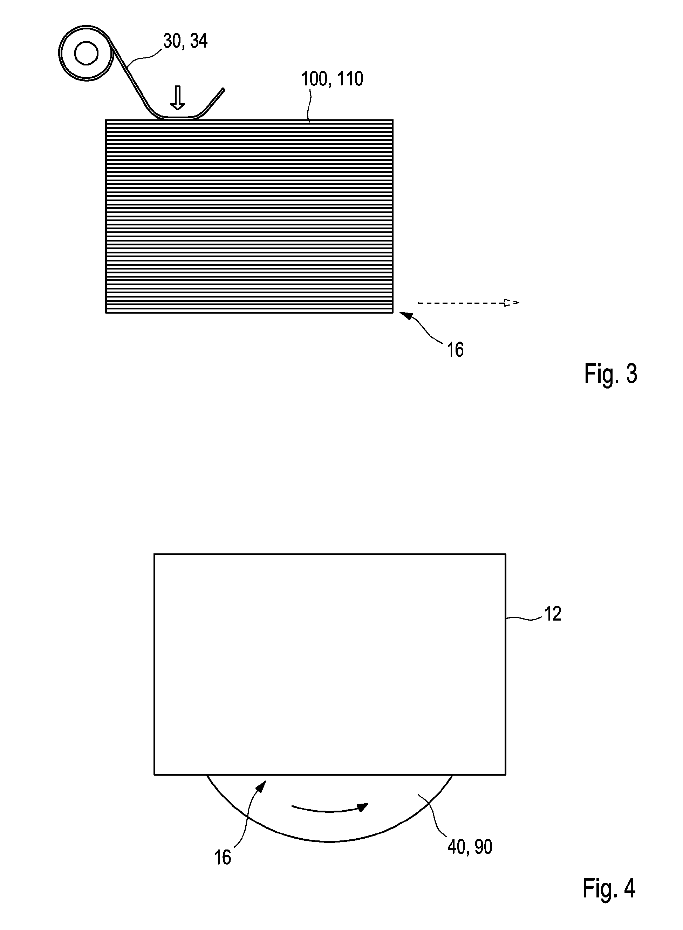

Figur 3 zeigt eine schematische Darstellung einer Mehrzahl von Elementen 100 in einem Stapel 110 mit einem Niederhalter 30 in Form einer Niederhalte-Feder 34.
Figur 4 zeigt ein Ausführungsbeispiel der Erfindung in Draufsicht mit einem Drehteller 90, der Bestandteil der Transporteinheit 40 ist. Der Stapel 110 der Elemente 100 befindet sich oberhalb des Drehtellers 90. Bewegt sich der Drehteller in seine Drehrichtung, wird das unterste der Elemente 100 mitgenommen und gleitet unter den darüber liegenden Elementen 100 des Stapels 110 weg.

## Patentansprüche

1. Hygieneartikel- und/oder Pflegeartikel-Spender (10) für im Wesentlichen flache Elemente (100), umfassend ein Gehäuse (12), welches wenigstens einen Vorratsraum (14) für die Elemente (100) umschließt, sowie eine Ausgabeöffnung (16) im Gehäuse (12), durch welche die Elemente (100) einzeln entnehmbar sind, **dadurch gekennzeichnet, dass** eine Transporteinheit (40) vorgesehen und dazu ausgebildet ist, ein zu entnehmendes Element (100) zur Ausgabeöffnung (16) so zu transportieren, dass das zu entnehmende Element (100) im Benutzungsfall zumindest bereichsweise aus dem Gehäuse (12) ragt.

2. Spender nach Anspruch 1, **dadurch gekennzeichnet, dass** die Transporteinheit (40) ausgebildet ist, ein zu entnehmendes Element (100) an wenigstens einer Seite des Elements (100) durch Reibschluss Richtung Ausgabeöffnung (16) anzutreiben.

3. Spender nach Anspruch 1 oder 2, **dadurch gekennzeichnet, dass** das zu entnehmende Element (100) auf einer Oberfläche (62) der Transporteinheit (40) aufliegt und unter dem darüberliegenden Element (100) hindurch gleitet.

4. Spender nach einem der vorhergehenden Ansprüche, **dadurch gekennzeichnet, dass** die Transporteinheit (40) eine adhäsive Oberfläche (62) aufweist.

5. Spender nach einem der vorhergehenden Ansprüche, **dadurch gekennzeichnet, dass** die Transporteinheit (40) zum Antreiben eines zu entnehmenden Elements (100) eine oder mehrere Transportrollen (60) aufweist.

6. Spender nach einem der vorhergehenden Ansprüche, **dadurch gekennzeichnet, dass** die Transporteinheit (40) zum Antreiben eines zu entnehmenden Elements (100) ein Transportband (70) aufweist.

7. Spender nach einem der vorhergehenden Ansprüche, **dadurch gekennzeichnet, dass** die Transporteinheit (40) Formschlussmittel (80) aufweist, um ein zu entnehmendes Element (100) durch Formschluss anzutreiben.

8. Spender nach Anspruch 7, **dadurch gekennzeichnet, dass** die Transporteinheit (40) zum Antreiben eines zu entnehmenden Elements (100) eine oder mehrere Spitzen (82) aufweist.

9. Spender nach einem der vorhergehenden Ansprüche, **dadurch gekennzeichnet, dass** die Transporteinheit (40) zum Antreiben eines zu entnehmenden Elements (100) einen Drehteller (90) aufweist.

10. Spender nach einem der vorhergehenden Ansprüche, **dadurch gekennzeichnet, dass** die Transporteinheit (40) elektromotorisch antreibbar ist.

11. Spender nach einem der vorhergehenden Ansprüche, **dadurch gekennzeichnet, dass** die Transporteinheit (40) durch eine mechanisch betätigbare Hebelvorrichtung (50) antreibbar ist, vorzugsweise eine Transporttrolle (60) durch die Hebelvorrichtung (50) antreibbar ist.

12. Spender nach einem der vorhergehenden Ansprüche, **dadurch gekennzeichnet, dass** die Transporteinheit (40) eine Münzaufnahme aufweist, welche die Transporteinheit (40) direkt oder indirekt in Gang setzt oder sperrt.

13. Spender nach einem der vorhergehenden Ansprüche, **dadurch gekennzeichnet, dass** der Vorratsraum (14) zur Aufnahme eines Stapels (110) der Elemente (100) ausgebildet ist.

14. Spender nach einem der vorhergehenden Ansprüche, **dadurch gekennzeichnet, dass** der Vorratsraum (14) zur Aufnahme von Elementen (100) als Wicklung einer Mehrzahl von Elementen (100) ausgebildet ist.
